# EUROPEAN PATENT SPECIFICATION

(11) **EP 3 832 306 B1**
(45) Date of publication and mention of the grant of the patent: **07.02.2024**
(21) Application number: 19840526.8
(22) Date of filing: 26.07.2019
(51) Int. Cl.: G01N 33/53, C12M 1/34, G01N 33/543

(54) **METHOD FOR IMMUNOLOGICAL ANALYSIS OF (1->3)-ß-D-GLUCAN IN BIOLOGICAL SAMPLE, KIT FOR ANALYSIS OF (1->3)-ß-D-GLUCAN, AND ALKALI PRETREATMENT SOLUTION FOR BIOLOGICAL SAMPLE FOR USE IN METHOD FOR IMMUNOLOGICAL ANALYSIS OF (1->3)-ß-D-GLUCAN**
VERFAHREN ZUR IMMUNOLOGISCHEN ANALYSE VON (1->3)-BETA-D-GLUCAN IN BIOLOGISCHEN PROBEN, KIT ZUR ANALYSE VON (1->3)-BETA-D-GLUCAN UND ALKALIVORBEHANDLUNGSLÖSUNG FÜR BIOLOGISCHE PROBEN ZUR VERWENDUNG IN VERFAHREN ZUR IMMUNOLOGISCHEN ANALYSE VON (1->3)-BETA-D-GLUCAN
PROCÉDÉ D'ANALYSE IMMUNOLOGIQUE DE (1->3)-ß-D-GLUCANE DANS UN ÉCHANTILLON BIOLOGIQUE, KIT POUR L'ANALYSE DE (1->3)-ß-D-GLUCANE, ET SOLUTION DE PRÉTRAITEMENT ALCALINE POUR ÉCHANTILLON BIOLOGIQUE DESTINÉ À ÊTRE UTILISÉ DANS LE PROCÉDÉ D'ANALYSE IMMUNOLOGIQUE DE (1->3)-ß-D-GLUCANE

(30) Priority: 27.07.2018 JP 2018141178
(43) Date of publication of application: 09.06.2021
(73) Proprietor: Sekisui Medical Co., Ltd., Tokyo 103-0027 (JP)
(72) Inventor: SUNAMURA, Ei-ichiro, Tokyo 103-0027 (JP); YOTANI, Takuya, Tokyo 103-0027 (JP)
(74) Representative: Hoffmann Eitle
(86) International application number: PCT/JP2019/029353
(87) International publication number: WO 2020/022467

(56) References cited:
- WO-A1-2004/036222
- WO-A1-2010/107068
- JP-A- H0 670 796
- JP-A- H04 346 791
- JP-A- H07 128 337
- JP-A- H11 196 895
- JP-A- 2017 501 399
- JP-B2- 5 054 426
- Nollstadt K H ET AL: "Use of 3-1,3-Glucan-Specific Antibody To Study the Cyst Wall of Pneumocystis carinii and Effects of Pneumocandin Bo Analog L-733,560", , 1 January 1994 (1994-01-01), pages 2258-2265, XP55901035, Retrieved from the Internet: URL:https://www.ncbi.nlm.nih.gov/pmc/artic les/PMC284727/pdf/aac00374-0028.pdf [retrieved on 2022-03-14]

## Description

### TECHNICAL FIELD

The present invention relates to an immunoassay method for (1→3)-β-D-glucan (hereinafter sometimes abbreviated as BG) in a biological sample. The present invention also relates to an assay kit for BG in a biological sample and an alkali pretreatment solution for a biological sample for use in an immunoassay method for BG in a biological sample.

### BACKGROUND ART

β-D-glucan is a glucose polymer in which multiple glucose molecules are bound by β-bonds. A carbon atom at the 1-position of glucose can bind to each of 5 carbons of the other glucose, i.e. carbons at the 1-position, the 2-position, the 3-position, the 4-position, and the 6-position. It has been reported that a bonding combination of the 1-position and 3-position (1→3), the 1-position and 4-position (1→4), or the 1-position and 6-position (1→6) frequently occurs in natural β-D-glucan.

β-D-glucan is a cell-wall constituent component of fungi and is a characteristic substance not found in other microorganisms such as bacteria. Due to this feature, β-D-glucan assay has been used in the test for deep mycosis. Deep mycosis is a type of opportunistic infection with which a patient with immune dysfunction due to weakened resistance, and the patient falls into an extremely critical condition. Examples of typical causative fungi of deep mycosis include the genera Candida and Aspergillus. Since the presence of BG is common to the cell walls of these causative organisms, measurement of BG in body fluid has been used for auxiliary diagnosis of deep mycosis infections.

Currently, a Limulus reagent utilizing a protective reaction of horseshoe crab to BG is used for the examination of deep mycosis (Patent Documents 1 and 2). This Limulus reagent is approved as an in-vitro diagnostic. However, a method using this Limulus reagent requires blood of horseshoe crab, which is a natural resource, and is therefore costly to maintain a certain quality in addition to causing a concern of resource depletion. Another drawback of this method is that dispersion tends to occur since multiple steps are required for this technique.

In recent years, to eliminate the drawbacks of the Limulus reagent and measure BG more quickly and easily, it is attempted to measure BG by using an antibody recognizing BG (Patent Documents 3 and 4, Non-Patent Documents 1 to 3). However, the antibodies used in these methods were far inferior to the Limulus reagent in terms of BG measurement sensitivity. Therefore, an immunoassay method for measuring BG for clinical use, i.e., usable for any actual specimen such as plasma containing BG derived from a fungus, does not yet exist.

### CITATION LIST

### PATENT LITERATURE

Patent Document 1: Japanese Patent No. 5089375
Patent Document 2: Japanese Patent No. 3553656
Patent Document 3: Japanese Laid-Open Patent Publication No. 4-346791
Patent Document 4: Japanese Patent No. 5054426

### NON PATENT LITERATURE

Non-Patent Document 1: Use of beta-1,3-glucan-specific antibody to study the cyst wall of Pneumocystis carinii and effects of pneumocandin B0 analog L-733, 560. Antimicrobial Agents and Chemotherapy 1994 Oct; 38(10): 2258-2265
Non-Patent Document 2: Plasma (1→3)-β-D-glucan assay and immunohistochemical staining of (1→3)-β-D-glucan in the fungal cell walls using a novel horseshoe crab protein (T-GBP) that specifically binds to (1→3)-β-D-glucan. Journal of Clinical Laboratory Analysis 1997 Volume 11, Issue 2, 104-109
Non-Patent Document 3: A novel monoclonal antibody recognizing beta (1-3) glucans in intact cells of Candida and Cryptococcus. APMIS 2008 Oct; 116(10): 867-876

### SUMMARY OF INVENTION

### TECHNICAL PROBLEM

As described above, the conventional technique for measuring BG using an antibody recognizing BG is inferior to the Limulus reagent in terms of BG measurement sensitivity. Therefore, a BG assay method using an antibody having the BG detection sensitivity equivalent to that of the Limulus reagent and easy to operate has been desired.

### SOLUTION TO PROBLEM

The invention is defined in the appended claims.

The present inventors conducted intensive studies for solving the problem and produced an antibody specifically recognizing BG. Furthermore, the present inventors found that an immunoassay method for BG having a sensitivity equivalent to that of the Limulus reagent can be performed by pretreating a biological sample with an alkali solution, thereby completing the present invention. In particular, the conventional immunoassay method for BG using an antibody has a low sensitivity that is not at a clinically usable level. In the present invention, by combining a pretreatment of a biological sample with an alkali solution and a use of an anti-(1→3)-β-D-glucan monoclonal antibody specifically reacting with BG (hereinafter sometimes referred to as an anti-BG monoclonal antibody), BG in the biological sample can be analyzed with high sensitivity. In particular, while the conventional immunoassay method for BG using an antibody can detect BG present in nanograms in 1 mL of a biological sample, the immunoassay method of the present invention can detect BG present in picograms in 1 mL of a biological sample.

### ADVANTAGEOUS EFFECTS OF INVENTION

According to the present invention, the immunoassay method for BG in a biological sample having a sensitivity equivalent to that of a Limulus reagent can be performed. The present invention can provide an immunological assay kit for BG in a biological sample having a sensitivity equivalent to that of a Limulus reagent.

### BRIEF DESCRIPTION OF DRAWINGS

[Fig. 1] Fig. 1 is a graph showing an effect of the present invention due to pretreatment with an alkali solution as compared to a pretreatment with an acid solution or a pretreatment with heat.
[Fig. 2] Fig. 2 is a graph showing an effect of the present invention due to pretreatment with an alkali solution as compared to a pretreatment with an acid solution or a pretreatment with heat.
[Fig. 3] Fig. 3 is a graph showing an influence of a difference in pH on the effect of the present invention.
[Fig. 4] Fig. 4 is a graph showing a result of study on time of the pretreatment with an alkali solution.
[Fig. 5] Fig. 5 is a graph showing a result of study on temperature conditions during the pretreatment with an alkali solution.
[Fig. 6] Fig. 6 is a graph showing a result of study on a lower limit of detection of the immunoassay method for BG of the present invention.
[Fig. 7] Fig. 7 is a graph showing a correlation between the immunoassay method for BG of the present invention and a commercially available Limulus reagent.

### DESCRIPTION OF EMBODIMENTS

### [1] Immunoassay Method for (1→3)-β-D-Glucan in Biological Sample

### (Biological Sample)

Examples of a "biological sample" in the present invention include solid tissues and body fluids derived from living bodies (organisms), and body fluids are preferably used. The biological sample in the present invention is more preferably blood, serum, plasma, urine, saliva, sputum, tear fluid, otorrhea, or prostatic fluid, more preferably blood, serum, or plasma, further preferably blood, serum, or plasma of a subject suspected of having deep mycosis, most preferably blood, serum, or plasma of a subject suspected of having deep mycosis caused by causative fungi of the genus Candida and/or the genus Aspergillus. Examples of the living body or the subject include humans or animals (e.g., monkeys, dogs, cats, mice, guinea pigs, rats, hamsters, horses, bovines, pigs, birds, and fish), and are preferably humans.

### (Pretreatment)

The immunoassay method of the present invention includes a step of pretreating a biological sample with an alkali solution. A time for performing the pretreatment is not particularly limited and is within 30 seconds, within 1 minute, within 3 minutes, or within 5 minutes, for example. The pretreatment may be performed for 5 minutes or more. A heat treatment etc. can also be performed before or after the pretreatment with the alkali solution as long as the effects of the present invention can be obtained. The "pretreatment of a biological sample with an alkali solution" means that the alkali solution and the biological sample are brought into contact with each other.

### (Alkali Solution)

Although the alkali solution used in the pretreatment step of the present invention is not particularly limited as long as the effects of the present invention can be obtained, for example, an alkali metal hydroxide can be used. Sodium hydroxide (NaOH), potassium hydroxide (KOH), or lithium hydroxide (LiOH) is particularly preferable.

The pH of the alkali solution is 12 or more. Although the molar concentration of the alkali solution is not limited as long as the effects of the present invention can be obtained, for example, when KOH is used, the molar concentration is 1 mM or more, preferably 4.5 mM or more, more preferably 9 mM or more, further preferably 18 mM or more, further preferably 35 mM or more, and most preferably 50 mM or more.

### (Temperature of Alkali Solution)

The temperature of the alkali solution used in the pretreatment step of the present invention 4 to 40 °C.

The pH and temperature of the alkali solution used in the pretreatment step of the present invention are pH of 12 or more and a temperature of 4 to 40 °C.

### (Neutralization of Alkali Solution)

After the treatment of the biological sample with the alkali solution, the alkali solution is preferably neutralized. Although a solution used for neutralizing the alkali solution is not limited as long as the effects of the present invention can be obtained, examples thereof include Tris/HCl etc. The pH after the neutralization can appropriately be adjusted depending on an antibody to be used and a biological sample and can be adjusted to pH 6 to 9, pH 6.5 to 8.5, or pH 7 to 8, for example.

### ((1→3)-β-D-Glucan)

In this description, "(1→3)-β-D-glucan" and "BG" mean glucan in which carbon at the 1-position of glucose and carbon at the 3-position of other glucose are bonded in a β-type bonding form. BG has a unique structure called a triple helix structure. In this description, "(1→3)-β-D-glucan" and "BG" can include (1→3)(1→6)-β-D-glucans such as laminarin and lentinan in which glucose binds to the outer side chain of this triple helix structure in the (1→6) form. In this description, "(1→3) -β-D-glucan" and "BG" can include (1→3)(1→4)-β-D-glucans such as β-glucans derived from barley and lichenin including the (1→4) binding form in addition to the (1→3) binding form.

Examples of "(1→3)-β-D-glucan" or "BG" in the present invention can include laminarin, lentinan, pachyman, curdlan, laminaritetraose, paramylon, carboxymethylpachyman, carboxymethylcurdlan, and BG present on a cell wall of fungus causing deep mycosis (e.g., BG present on a cell wall of Aspergillus fungus, and BG present on a cell wall of Candida fungus).

As used herein, the "deep mycosis" means a condition in which a fungus entering a deep part of the body such as lungs, liver, kidneys, or brain causes infection. Deep mycosis mainly occurs in patients having undergone organ transplantation and receiving immunosuppressive drugs. Examples of the causative fungi of deep mycosis include Aspergillus fungi and Candida fungi, and the immunoassay method of the present invention can effectively analyze BG present on the cell walls of these fungi.

### (Anti-(1→3)β-D-Glucan Monoclonal Antibody)

The anti-(1→3)-β-D-glucan monoclonal antibody and the "anti-BG monoclonal antibody" used in the present invention specifically react with BG. "Specifically reacting with BG" means that the antibody reacts with BG and does not substantially react with a substance having a similar structure. The meaning of "not substantially reacting" will be described later. Specific examples of the anti-BG monoclonal antibody used in the present invention include monoclonal antibodies 86202R, 86207, and 86208.

Although "reacting" with BG, "recognizing" BG, and "binding" to BG are synonymously used in this description, they must be construed in the broadest sense without being limited to these exemplifications. Whether an antibody "reacts" with an antigen (compound) such as BG can be confirmed by an antigen-immobilized ELISA method, a competitive ELISA method, a sandwich ELISA method, etc. as well as by using the principle of surface plasmon (SPR method) etc. The SPR method can be performed by using devices, sensors, and reagents commercially available under the name of Biacore (registered trademark).

Stating that the antibody used in the present invention "does not react with" a certain compound means that the antibody used in the present invention does not substantially react with a certain compound, while stating "not substantially reacting" means that enhanced reactivity of the antibody used in the present invention is not recognized when Biacore (registered trademark) T100 or T200 is used for immobilizing and measuring the antibody of the present invention based on the SPR method, for example. Specifically, this means that the reactivity between the antibody and the compound is not significantly different from the reactivity of a control (with no compound added). Obviously, it can be confirmed that the antibody "does not substantially react" with the compound by a method or means well known to those skilled in the art, in addition to the SPR method.

The anti-BG monoclonal antibody used in the immunoassay method of the present invention includes a fragment having a function of the monoclonal antibody as long as the effects of the present invention can be obtained. Examples of the fragment include a functional fragment containing the Fab portion of the monoclonal antibody obtained by enzymatic digestion of the anti-BG monoclonal antibody, a functional fragment containing the Fab portion of the monoclonal antibody prepared by gene recombination, and a functional fragment containing scFv prepared by a phage display method, etc.

The antibody used in the immunoassay method of the present invention can be produced by dissolving BG such as laminariheptaose as an antigen (immunogen) in a solvent such as phosphate buffered saline and administering this solution to an animal for immunization. The immunization may be performed using an emulsion after adding an appropriate adjuvant to the solution as required. The adjuvant may be a widely used adjuvant, such as water-in-oil emulsion, water-in-oil-in-water emulsion, oil-in-water emulsion, liposome, or aluminum hydroxide gel as well as a protein or peptidic substance derived from biogenic components. For example, Freund's incomplete or complete adjuvant can preferably be used. Although not particularly limited, it is desired that the administration route, administered dose, and administration time of the adjuvant are appropriately selected such that a desired immune response can be enhanced in an animal to be immunized by the antigen.

Although not particularly limited, the type of the animal used for the immunization is preferably a mammal and can be a mouse, rat, bovine, rabbit, goat, sheep, and alpaca, and a mouse or rat is more preferable. The animal may be immunized in accordance with a common technique, e.g., the immunization can be achieved by subcutaneously, intracutaneously, intravenously, or intraperitoneally injecting the animal with a solution of an antigen, preferably a mixture with the adjuvant. Since an immune response is generally different depending on a type and strain of the animal to be immunized, it is desirable that an immunization schedule is appropriately set depending on the animal to be used. The antigen administration is preferably repeated several times after initial immunization.

Although the following operations are continuously performed so as to obtain a monoclonal antibody, the operation is not limited thereto, and a method of producing the monoclonal antibody itself is well known in the art and is widely used, so that the antibody used in the immunoassay method of the present invention can easily be produced by using the antigen described above (see, e.g., Antibodies, A Laboratory Manual (Cold Spring Harbor Laboratory Press, (1988), Chapter 6)).

After final immunization, a hybridoma can be produced by extracting spleen or lymph node cells, which are antibody-producing cells, from an immunized animal and by fusing the cells with a myeloma-derived cell line having high proliferative ability. Cells having high antibody-producing ability (quantitative and qualitative) are preferably used for the cell fusion, and the myeloma-derived cell line is preferably compatible with the animal from which the antibody-producing cells to be fused are derived. The cell fusion can be performed in accordance with a method known in the art, and a polyethylene glycol method, a method using Sendai virus, or a method utilizing electric current can be employed. The obtained hybridoma can be proliferated in accordance with a known method, and the desired hybridoma can be selected while confirming a property of a produced antibody. The hybridoma can be cloned by a well-known method such as a limiting dilution or soft agar method.

The hybridoma can efficiently be selected at the selection stage, considering the conditions under which the produced antibody is actually used in the measurement. For example, an antibody obtained by immunizing an animal is reacted with BG immobilized on a solid phase in the presence of a compound for which cross-reactivity is desired to be confirmed, and the reactivity can be compared with that in the absence of the compound for which cross-reactivity is desired to be confirmed so as to more efficiently select the hybridoma producing a desired antibody. Alternatively, an antibody obtained by immunizing an animal is reacted with BG immobilized on a solid phase in the presence of a biological sample-derived component, and the reactivity can be compared with that in the absence of the biological sample-derived component so as to more efficiently select the hybridoma producing a desired antibody.

After a cloning step, the binding ability of the produced antibody to BG can be assayed by using a method such as an ELISA method, an RIA method, and a fluorescent antibody method so as to confirm whether the selected hybridoma produces a monoclonal antibody having a desired property.

A monoclonal antibody having a desired property can be produced by the mass cultivation of the hybridoma selected as described above. Although a method of mass cultivation is not particularly limited, examples thereof can include a method of producing the monoclonal antibody in culture media by cultivating the hybridoma in appropriate culture media and a method of producing the antibody in abdominal dropsy by injecting the hybridoma into the abdominal cavity of a mammal for proliferation. The monoclonal antibody can be purified by appropriately combining the methods for purifying an antibody from the antiserum described above, for example, DEAE anion exchange chromatography, affinity chromatography, an ammonium sulphate fractionation method, a PEG fractionation method, and an ethanol fractionation method.

The antibody used in the immunoassay method of the present invention can be a whole antibody molecule as well as a fragment of an antibody having an antigen-antibody reaction activity and can be an antibody obtained through an immunization step of an animal as described above or obtained by a gene recombination technique, or a chimeric antibody. The fragment of the antibody is preferably a functional fragment; examples thereof include F(ab')₂, Fab', scFv, etc.; and these fragments can be produced by processing the antibody obtained as described above with a proteolytic enzyme (e.g., pepsin or papain), or by cloning of DNA of the antibody and expression in a culture system using Escherichia coli or yeast.

The antibody used in the immunoassay method of the present invention can be used as an immobilized (solid-phased) antibody immobilized on an insoluble carrier or as a labeled antibody labeled with a labeling substance well known to those skilled in the art described later. For example, the immobilized antibody can be produced by causing an insoluble carrier to physically adsorb or chemically bind to a monoclonal antibody (a suitable spacer may exist therebetween). The insoluble carrier can be made of a polymer base material such as a polystyrene resin, an inorganic base material such as glass, and a polysaccharide base material such as cellulose and agarose, and the shape thereof is not particularly limited and can be selected from any shapes such as a plate shape (e.g., microplate and membrane), a bead or particle shape (e.g., latex particles and colloidal magnetic particles), and a tubular shape (e.g., test tube).

By using a labeled antibody (secondary antibody) that can bind to the antibody used in the immunoassay method of the present invention, an amount of the antibody bound to BG can be measured, and BG in a biological sample can thereby be detected. Examples of the labeling substance for producing the labeled antibody include enzymes, fluorescent substances, chemiluminescent substances, biotin, avidin, radioisotopes, colloidal gold particles, or colored latex. A method for binding the labeling substance and the antibody can be a method such as a glutaraldehyde method, a maleimide method, a pyridyl disulfide method, or a periodic acid method, which can be used by those skilled in the art, and the types of the immobilized and labeled antibodies and the methods for producing the antibodies are not limited to these examples. For example, when an enzyme such as horseradish peroxidase (HRP) or alkali phosphatase (ALP) is used as the labeling substance, the enzyme activity can be measured by using a specific substrate of the enzyme (e.g., O-phenylenediamine (OPD) or 3,3',5,5'-tetramethylbenzidine (TMB) when the enzyme is HRP, p-nitrophenyl phosphate in the case of ALP), and when biotin is used as the labeling substance, at least avidin or enzyme-modified avidin is typically reacted therewith. In the immunoassay method of the present invention, biotin or HRP is preferably used as the labeling substance.

In this description, an "insoluble carrier" may be represented as a "solid phase", and physically or chemically supporting an antigen or antibody with an insoluble carrier or the supporting state may be represented as "immobilizing", "immobilized", or "solid phased". The term "detection" or "measurement" must be construed in the broadest sense, including the existence proof and/or the quantitation of BG and must not be construed in a limited manner in any sense.

### (Immunoassay Method)

Examples of the immunoassay method of the present invention include but not limited to ELISA, enzyme immunoassay, an immunohistochemical staining method, a surface plasmon resonance method, latex agglutination immunoassay, chemiluminescent immunoassay, electrochemiluminescence immunoassay, a fluorescent antibody method, radioimmunassay, an immunoprecipitation method, a Western Blot method, immunochromatography, high-performance liquid chromatography (HPLC), etc. It is preferable to use ELISA as the immunoassay method of the present invention. Among ELISAs, a sandwich ELISA using a first anti-BG monoclonal antibody immobilized on a solid phase and a labeled second anti-BG monoclonal antibody is preferable. In this case, the 86207 antibody can be used as the first anti-BG monoclonal antibody, and the 86202R antibody can be used as the second anti-BG monoclonal antibody. The same antibody may be used as the first anti-BG monoclonal antibody and the second anti-BG monoclonal antibody. The antibody immobilized on the solid phase or the labeled antibody may be a mixture of multiple antibodies. Biotin or HRP is preferably used as the labeling substance for the labeled antibody.

### (Diagnosis, Diagnostic assistance, and Treatment of Deep Mycosis)

Based on an analysis result of the immunoassay method of the present invention, a diagnosis can be made on whether the subject has deep mycosis, or the diagnosis can be assisted. The sensitivity of the conventional immunoassay method using an antibody is insufficient, and a patient with deep mycosis in the early stage of the disease may have a negative result. Early detection and early treatment are important for deep mycosis. By using the highly-sensitive immunoassay method of the present invention, early detection and early treatment of deep mycosis can be achieved. Not part of the invention is that after performing the immunoassay method of the present invention, if necessary, based on a result of a step of assaying BG in a biological sample, another method for assaying deep mycosis may be implemented for the patient, and/or a drug for the treatment of deep mycosis may be administered to the patient.

The immunoassay method can include a step of detecting 6 pg/mL or more of BG contained in a biological sample, and/or a step of diagnosing or an auxiliary step of diagnosing a subject corresponding to the collected biological sample as having or being suspected of having deep mycosis, based on the result of the detection step. A cutoff value can appropriately be set depending on a type of the biological sample or a type of the immunoassay method. For example, the immunoassay method of the present invention can include a step of detecting 7 pg/mL or more, 8 pg/mL or more, 9 pg/mL or more, 10 pg/mL or more, 11 pg/mL or more, 20 pg/mL or more, 50 pg/mL or more, 80 pg/mL or more, or 100 pg/mL or more of BG contained in a biological sample, and/or a step of diagnosing or an auxiliary step of diagnosing a subject corresponding to the collected biological sample as having or being suspected of having deep mycosis, based on the result of the detection step. The subject corresponding to the collected biological sample can be a subject to which an anticancer agent or an immunosuppressive agent is administered.

### [2] Assay Kit for (1→3)-β-D-Glucan in Biological Sample

A kit provided by the present invention contains (a) a solid phase such as a plate on which a first anti-BG antibody is immobilized, (b) an alkali pretreatment solution for a biological sample, (c) a solution for neutralization of the alkali pretreatment solution and preferably includes (d) a second anti-BG monoclonal antibody labeled with a labeling substance. The solid phase having the first anti-BG monoclonal antibody immobilized thereon captures BG in a biological sample to form a BG-antibody complex. The second anti-BG monoclonal antibody labeled with the labeling substance reacts with this BG-antibody complex to form a sandwich. The BG in the sample can be measured by measuring an amount of the labeling substance by a method corresponding to the labeling substance. For specific methods for constructing the kit, such as a method for immobilizing the first anti-BG monoclonal antibody on the solid phase and a method for labeling the second anti-BG monoclonal antibody with a labeling substance, the methods described in this description and the methods well known to those skilled in the art can be used without limitation.

The first anti-BG monoclonal antibody and the second anti-BG monoclonal antibody are not particularly limited as long as the antibodies are anti-BG monoclonal antibodies specifically reacting with BG. For example, the 86207 antibody can be used as the first anti-BG monoclonal antibody, and the 86202R antibody can be used as the second anti-BG monoclonal antibody.

For the labeling substance, for example, a labeling substance known to those skilled in the art such as a fluorescent substance, a chemiluminescent substance, biotin, and avidin can be used. A method for binding the labeling substance and the antibody can appropriately be selected from known binding methods depending on the labeling substance and antibody to be used and can be, for example, a method such as a glutaraldehyde method, a maleimide method, a pyridyl disulfide method, or a periodic acid method. Biotin or HRP is preferably used as the labeling substance.

The kit of the present invention can also contain an instruction manual etc. The kit may contain any constituent element, such as a buffer, a stabilizer, a reaction vessel, etc. Examples of (b) the alkali pretreatment solution for biological sample include an alkali specimen extract solution and an alkali specimen diluent.

The kit of the present invention can detect 6 pg/mL or more of BG contained in a biological sample, and based on this result, the subject corresponding to the collected biological sample can be diagnosed as having or being suspected of having deep mycosis. The cutoff value can appropriately be set depending on a type of the biological sample etc. For example, the kit of the present invention can detect 7 pg/mL or more, 8 pg/mL or more, 9 pg/mL or more, 10 pg/mL or more, 11 pg/mL or more, 20 pg/mL or more, 50 pg/mL or more, 80 pg/mL or more, or 100 pg/mL or more of BG contained in a biological sample, and based on this result, the subject corresponding to the collected biological sample can be diagnosed as having or being suspected of having deep mycosis.

The present invention will hereinafter specifically be described with examples; however, these examples do not limit the scope of the present invention.

### EXAMPLES

### [Test Example 1] Method for Producing Monoclonal Antibody Used in the Present Invention.

### 1. Preparation of Immunizing Antigen

Laminariheptaose (manufactured by Seikagaku Biobusiness Corporation) is BG having a structure with 7 glucoses polymerized in a linear shape and was used as an immunizing antigen. A laminariheptaose-transferrin conjugate was prepared by the same preparation method as described in Non-Patent Document 1 and used as an immunizing antigen.

### 2. Production of Hybridoma and Collection of Antibody

The whole amount of a suspension composed of 100 µl of solution of the prepared laminariheptaose-transferase conjugate (1 mg/mL), 0.25 mL of PBS (pH 7.2), and 0.25 mL of Freund's adjuvant (complete adjuvant or complete adjuvant) was administered subcutaneously to the back of, or intraperitoneally to, each of a BALB/c mouse (female) and an F344/Jc1 rat (female) 3 to 6 times in total. An administration interval was 2 weeks, and Freund's complete adjuvant was used for the first administration, while Freund's incomplete adjuvant was used for the latter four administrations. One week after the fifth administration, the abdominal cavities of the mouse and the rat were opened to remove their spleens, and single cells were obtained by pipetting.

These splenocytes were washed twice with PRMI1640 medium with no serum added and were mixed with separately cultured and washed mouse myeloma cells (X-63-Ag8-6.5.3) at a ratio of 1 × 10⁷ myeloma cells to 5×10⁷ splenocytes, and the mixture was centrifuged to remove the supernatant. The sediment was well dissolved, polyethylene glycol 1540 (1 mL) serving as a fusion accelerator was slowly added at 37 °C for 1 minute, and the mixture was further stirred for 1 minute for fusion. After these fused cells (hybridomas) were suspended in 10 mL of RPMI1640 medium supplemented with fetal bovine serum and were centrifuged, the residue was seeded on a single 96-well culture plate and cultured in a 5% CO₂ incubator at 37 °C for 1 week. After culturing in HAT medium at 37 °C for 1 week, only hybridomas were selectively collected. The culture supernatants thereof were collected to perform ELISA using laminarin, which is a type of BG, as an antigen, and three highly reactive hybridomas (rat-derived: 86202R, mouse-derived: 86207 and 86208) were selected and cloned. Ascites was collected from mice intraperitoneally injected with each hybridoma cell and cryopreserved at -80 °C. Each antibody was purified from the cryopreserved ascites by using a protein A or protein G column.

### [Example 1: Confirmation of Influence of Difference in Pretreatment (Alkali, Acid, or Heat) on BG Measurement Sensitivity in Specimen]

In Example 1, sandwich ELISA was performed by using a human plasma specimen to examine an influence of pretreatment with alkali, pretreatment with acid, or pretreatment with heat on BG measurement sensitivity.

The 86207 antibody was used as a solid-phase antibody, and the biotin-labeled 86202R antibody was used as a liquid-phase antibody.

### 1-1. Pretreatment with Alkali

To 44 µL of a human plasma specimen, 77.4 µL of alkali pretreatment solution (150 mM KOH: potassium hydroxide) was added and incubated at 37 °C for 15 minutes. Subsequently, 98.6 µL of 1 M Tris/HCl (pH 7.5) was added to the alkali pretreatment solution to neutralize the alkali pretreatment solution (220 µL in total; 5-fold dilution of the specimen; pH after neutralization was less than 7.9). This was used as an alkalipretreated specimen.

To 77.4 µL of the alkali pretreatment solution, 98.6 µL of 1 M Tris/HCl (pH 7.5) was added in advance to neutralize the solution. Subsequently, 44 µL of a human plasma specimen was added to obtain a specimen having the same solution composition and not subjected to alkali pretreatment.

### 1-2. Pretreatment with Acid (Perchloric Acid)

To 80 µL of a human plasma specimen, 80 µL of 2.5% perchloric acid was added and incubated at 37 °C for 15 minutes (white precipitate was generated). Subsequently, the incubated specimen was centrifuged (14000 rpm, 15 minutes, 4 °C) to collect 88 µL of the supernatant, and 138 µL of 1 M Tris/Cl (pH 7.5) was added to neutralize the perchloric acid (220 µL in total; 5-fold dilution of the specimen; pH after neutralization was less than 7.4). The specimen was returned to room temperature and then used as an acid-pretreated specimen.

To 44 µL of 2.5% perchloric acid, 138 µL of 1 M Tris/Cl (pH 7.5) was added for neutralization in advance. Subsequently, 44 µL of a human plasma specimen was added to obtain a specimen having the same solution composition and not pretreated with perchloric acid.

### 1-3. Pretreatment with Heat

To 44 µL of a human plasma specimen, 176 µL of PBS was added (220 µL in total; 5-fold dilution of specimen) and incubated at 75 °C for 15 minutes. The incubated specimen was returned to room temperature and then used as a heat-pretreated specimen.

The diluted human plasma specimen was incubated at room temperature (20 to 25 °C) for 15 minutes to obtain a specimen having the same solution composition and not treated with heat.

### 1-4. Specimen Amount

For each specimen, a specimen amount for two measurements (220 µL; 100 µL was used for one measurement) were prepared and used for sandwich ELISA.

### 1-5. Biotin Labeling of Antibodies

The 86202R antibody was adjusted to 2 mg/mL (13.7 µM, diluted with PBS) and reacted with a 20-fold amount (274 µM) of EZ-Link Sulfo-NHS-LC-Biotin (manufactured by Thermo Fisher Scientific). The reaction was performed on ice for 2 hours.

By dialysis with PBS, unreacted EZ-Link Sulfo-NHS-LC-Biotin was removed. The dialysis was performed twice at 4 °C in 100-fold amount (using Slide-A-Lyzer Diarysis Cassettes 10k (manufactured by Thermo Fisher Scientific)).

After dialysis, an antibody concentration was determined from absorbance by using a spectrophotometer to obtain the biotin-labeled 86202R antibody.

### 1-6. Sandwich ELISA

The 86207 antibody (5 µg/mL, 100 µL, diluted with PBS) for the solid phase was dispensed into each well of a 96-well plate (nunc immunoplate, part number 442404, manufactured by Thermo Fisher Scientific) and allowed to stand overnight at 4 °C.

After removing liquid from each well, 200 µL of PBST was dispensed into each plate well twice by using an 8-channel Pipetman (400 µL in total). The added PBST was removed, and these operations were defined as one wash operation. This operation was performed three times (washing with 400 µL of PBST three times; the same procedure was performed for washing in the following operations).

After washing, 200 µL of a blocking solution was dispensed into each well and allowed to stand at room temperature for 1 hour or more. After discarding the blocking solution, 100 µL of each of the pretreated specimens was added to each well and reacted for 2 hours.

After the reaction, the removal of the liquid in each well was followed by washing with 400 µL of PBST three times.

The biotin-labeled 86202R antibody (1 µg/mL, 100 µL, diluted with blocking solution) prepared in 1-5 was dispensed into each well and reacted for 2 hours.

After the reaction, the removal of the liquid in each well was followed by washing with 400 µL of PBST three times.

Into each well, 100 µL of Streptavidin Protein HRP Conjugate (Part No. 21126, manufactured by Thermo Fisher Scientific) diluted to 0.5 µg/mL with the blocking solution was dispensed and allowed to stand for 1 hour.

Into each well, 100 µL of a coloring solution was dispensed and reacted at room temperature for 10 minutes.

Into each well, 100 µL of a reaction stop solution was added.

The absorbance at 490 nm was measured with a microplate reader (iMark manufactured by Bio-Rad was used).

### 1-7. Results

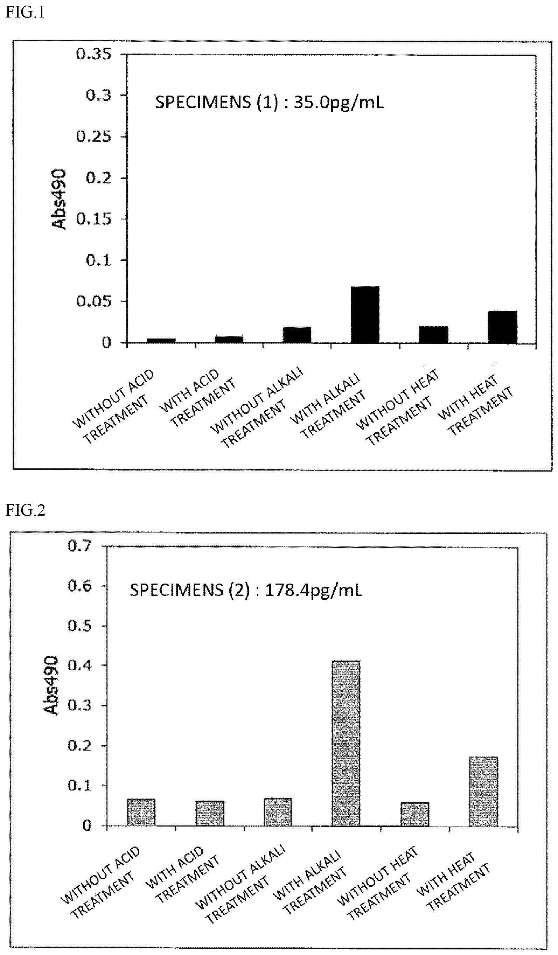

Fig. 1 shows measurement results of specimens containing 35.0 pg/mL of BG (measured by β-Glucan Test Wako: Limulus reagent manufactured by Wako Pure Chemical Industries, Ltd.). Fig. 2 shows the measurement results of the specimens containing 178.4 pg/mL of BG (measured by β-Glucan Test Wako).

In either type of the specimens containing 35.0 pg/mL or 178.4 pg/mL of BG, the measurement sensitivity was significantly improved in the specimen subjected to the alkali pretreatment as compared to the specimens subjected to the other two types of pretreatment (acid and heat).

### [Example 2: Confirmation of Influence of Difference in pH of Pretreatment Solution on BG Measurement Sensitivity in Specimen]

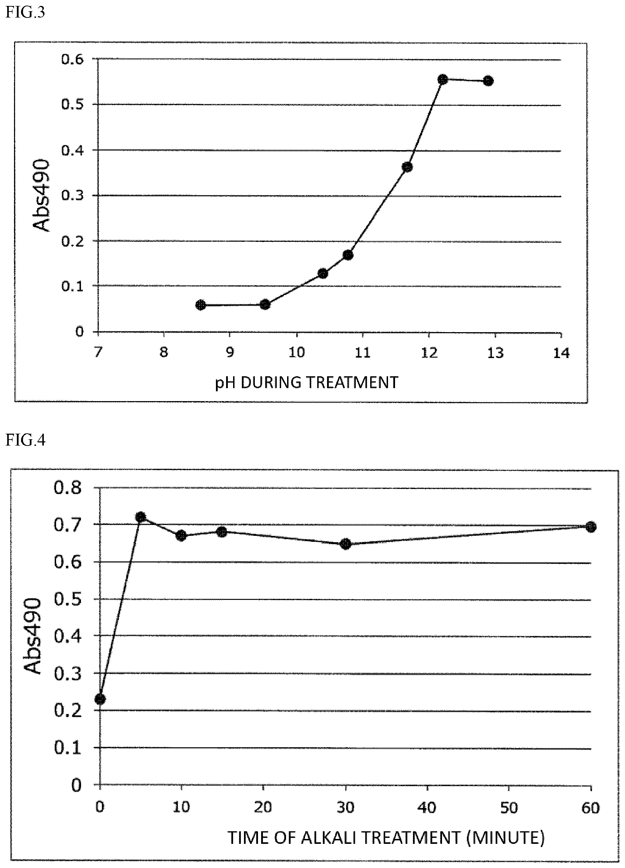

In Example 2, human serum specimens were pretreated with alkali solutions having various pH. By using a specimen obtained by adding CM-pachyman (carboxymethyl pachyman: a type of BG, manufactured by Megazyme) to negative serum containing no BG derived from fungus, an influence of a pH value on sensitivity of BG measurement in human serum specimens was examined. When the Limulus reagent (β-Glucan Test Wako: manufactured by Wako Pure Chemical Industries, Ltd.) is used, a specimen obtained by adding CM-pachyman shows reactivity close to that of an actual specimen containing BG derived from fungus. The absorbance of the specimen obtained by adding 6 ng/mL CM-pachyman corresponds to the absorbance of the actual specimen containing 174 pg/mL BG derived from fungus. The procedure for the pretreatment with an alkali solution, the solid phase and liquid phase antibodies used, the biotin labeling of the antibodies and sandwich ELISA were the same as Example 1. The concentration of a KOH solution for pretreatment used, the pH during pretreatment, the pH after neutralization, and the measured absorbance value (absorbance after subtracting a blank value) are as shown in Table 1 below. A relationship between pH and absorbance during treatment is shown in Fig. 3.

**[Table 1]**

| KOH concentration (mM) | pH during treatment | pH after neutralization | 6 ng/mL CM-pachyman (corresponding to 174 pg/mL BG in actual specimen) |
|---|---|---|---|
| 0.0 | 8.56 | 7.50 | 0.058 |
| 4.7 | 9.53 | 7.53 | 0.059 |
| 9.4 | 10.40 | 7.60 | 0.129 |
| 18.8 | 10.78 | 7.67 | 0.170 |
| 37.5 | 11.68 | 7.66 | 0.364 |
| 75.0 | 12.21 | 7.74 | 0.557 |
| 150.0 | 12.90 | 7.88 | 0.554 |

When the pH exceeded 10, the sensitivity increased, and when the pH is 12.2 or more, the increase in sensitivity peaked.

### [Example 3: Confirmation of Influence of Alkali Pretreatment Time on BG Measurement Sensitivity in Specimen]

In Example 3, an influence of a time of pretreatment of a specimen with an alkali solution on sensitivity of BG measurement was examined. The pretreatment procedure was the same as the pretreatment with alkali in Example 1 except that the pretreatment time was changed to various times and a human serum specimen obtained by adding CM-pachyman was used. The procedures for the biotin labeling of the antibodies and sandwich ELISA were the same as Example 1. The results are shown in Table 2 and Fig. 4. The measured absorbance value represents the absorbance after subtracting a blank value.

**[Table 2]**

| Treatment time (minute) | 6 ng/mL CM-pachyman (corresponding to 174 pg/mL in actual specimen) |
|---|---|
| 0 | 0.230 |
| 5 | 0.720 |
| 10 | 0.670 |
| 15 | 0.682 |
| 30 | 0.649 |
| 60 | 0.698 |

It was found that even when the treatment time was changed to 5 minutes, 10 minutes, 15 minutes, 30 minutes, and 60 minutes, the measurement sensitivity of BG in the specimen was hardly affected.

### [Example 4: Confirmation of Influence of Alkali Pretreatment Temperature on BG Measurement Sensitivity in Specimen]

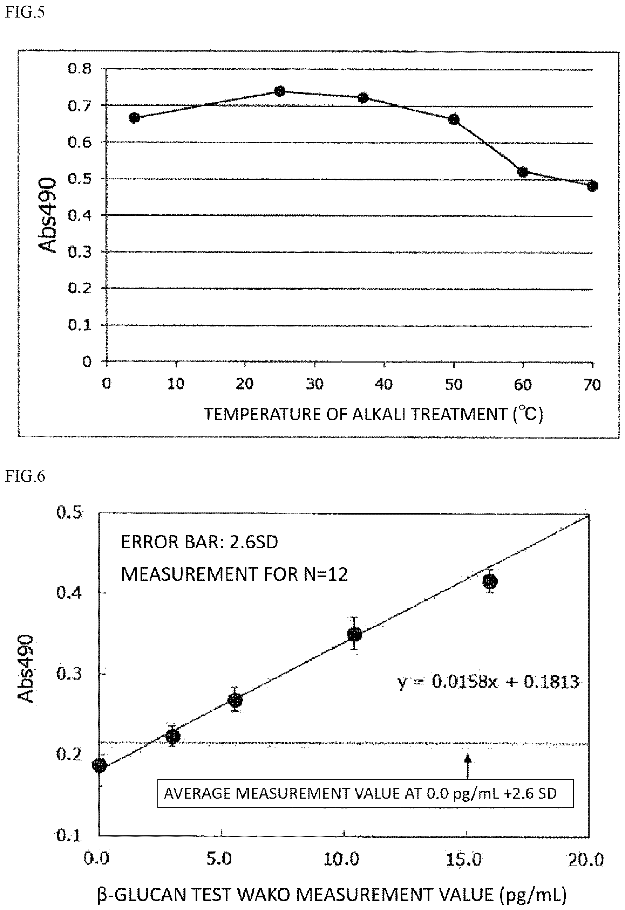

In Example 4, an influence of a temperature during the alkali pretreatment on sensitivity of BG measurement was examined. The pretreatment procedure was the same as the alkali pretreatment in Example 1 except that the temperature during the pretreatment was changed to various temperatures and a human serum specimen obtained by adding CM-pachyman was used. The procedures for the biotin labeling of the antibodies and sandwich ELISA were the same as Example 1. The results are shown in Table 3 and Fig. 5.

**[Table 3]**

| Treatment temperature (°C) | 6 ng/mL CM-pachyman (corresponding to 174 pg/mL in actual specimen) |
|---|---|
| 4 | 0.667 |
| 25 | 0.741 |
| 37 | 0.724 |
| 50 | 0.665 |
| 60 | 0.523 |
| 70 | 0.484 |

It was found that the sensitivity becomes best when the temperature was 4 to 37°C.

### [Example 5: Examination of Lower Limit of Detection of BG in Actual Specimen]

In Example 5, the lower limit of detection of sandwich ELISA was examined by using an actual specimen. The 86207 antibody was used as the solid-phase antibody, and the biotin-labeled 86202R antibody was used as the liquid-phase antibody.

### 5-1. Pretreatment with Alkali

An actual specimen (human plasma containing 421.2 pg/mL BG derived from fungus, measured by β-Glucan Test Wako) was diluted in stages by using a pooled plasma for dilution acquired by mixing 12 BG-negative specimens to prepare a dilution series. To 1020 µL of the specimen, 168 µL of the alkali treatment solution (800 mM KOH: potassium hydroxide) was added and incubated at 37 °C for 15 minutes. Subsequently, 168 µL each of 800 mM HCl and 100 mM MOPS (pH 7.5) were added to neutralize the alkali treatment solution (1356 µL in total, 3/4-fold dilution of the specimen, for 12 measurements). These specimens were used as alkali pretreated specimens.

### 5-2. Biotin Labeling of Antibodies

The antibodies were labeled with biotin in the same procedure as Example 1.

### 5-3. Sandwich ELISA

Sandwich ELISA was performed in the same procedure as Example 1. However, the concentration of the biotin-labeled antibody was 4 µg/mL. Each sample was measured for n=12.

### 5-4. Results

The results are shown in Table 4 and Fig. 6. The absorbance in Table 4 represents an average value of measurement for n=12, and SD represents a standard deviation.

**[Table 4]**

| Actual density (pg/mL) | Absorbance (average value of n=12) | SD | 2.6SD | Average value ±2.6 SD |
|---|---|---|---|---|
| 0.0 | 0.187 | 0.0103 | 0.027 | 0.214 |
| 3.0 | 0.223 | 0.0047 | 0.012 | 0.211 |
| 5.5 | 0.269 | 0.0057 | 0.015 | 0.254 |
| 10.4 | 0.351 | 0.0079 | 0.021 | 0.330 |
| 15.9 | 0.417 | 0.0054 | 0.014 | 0.403 |
| 20.6 | 0.518 | 0.0057 | 0.015 | 0.503 |

The value (0.214) obtained by adding 2.6 SD of the specimen to the average value of the absorbance of the specimen having an actual density of 0.0 pg/mL is smaller than the value (0.254) obtained by subtracting 2.6 SD of the specimen of 5.5 pg/mL from the average absorbance of the specimen, and therefore, it was found that even when BG derived from fungus contained in the actual specimen is 5.5 pg/mL (the measurement value of the Limulus reagent), BG can be detected in ELISA serving as an embodiment of the immunoassay method of the present invention. The cutoff value of the Limulus reagent manufactured by Wako Pure Chemical Industries, Ltd. is 11 pg/mL, and this result indicated that ELISA serving as an embodiment of the immunoassay method of the present invention has the sensitivity equivalent to that of the Limulus reagent.

### [Example 6: Examination of Correlation with Commercially Available Limulus Reagent]

In Example 6, the correlation between the measurement value of sandwich ELISA and the measurement value of the Limulus reagent was examined by using a human plasma specimen (n=39). The 86207 antibody was used as the solid-phase antibody, and the HRP-labeled 86202R antibody was used as the liquid-phase antibody.

### 6-1. Pretreatment with Alkali

The procedure was the same as Example 1 except that human plasma was used.

### 6-2. HRP Labeling of Antibodies

HRP labeling of the 86202R antibody was performed by using an HRP labeling kit (Peroxidase Labeling Kit-SH, LK09, manufactured by Dojindo Laboratories).

Protein quantification after labeling was performed by a BCA method (kit name: Micro BCA protein assay kit, manufactured by Thermo Scientific, part number: 23235).

### 6-3. Sandwich ELISA

The 86207 antibody (5 µg/mL, 100 µL, diluted with PBS) for the solid phase was dispensed into each well of a 96-well plate (nunc immunoplate, part number 442404, manufactured by Thermo Fisher Scientific) and allowed to stand overnight at 4 °C.

After removing liquid from each well, 200 µL of PBST was dispensed into each plate well twice by using an 8-channel Pipetman (400 µL in total). The added PBST was removed, and these operations were defined as one wash operation. This operation was performed three times (washing with 400 µL of PBST three times; the same procedure was performed for washing in the following operations).

After washing, 200 µL of a blocking solution was dispensed into each well and allowed to stand at room temperature for 1 hour or more.

After discarding the blocking solution, 100 µL of each of the pretreated specimens was added to each well and reacted for 2 hours.

After the reaction, the removal of the liquid in each well was followed by washing with 400 µL of PBST three times.

The HRP-labeled 86202R antibody (0.5 µg/mL, 100 µL, diluted with the blocking solution) prepared in 6-2 was dispensed into each well and reacted for 30 minutes.

After the reaction, the removal of the liquid in each well was followed by washing with 400 µL of PBST three times.

Into each well, 100 µL of a coloring solution was dispensed and reacted at room temperature for 10 minutes.

Into each well, 100 µL of a reaction stop solution was added.

The absorbance at 492 nm was measured with a microplate reader (Multiskan FC, manufactured by Thermo Fisher Scientific).

### 6-4. Limulus Reagent

BG was measured in accordance with the protocol described in the package insert of the Limulus Reagent (trade name: β-Glucan Test Wako, manufactured by Wako Pure Chemical Industries, Ltd.).

### 6-5. Results

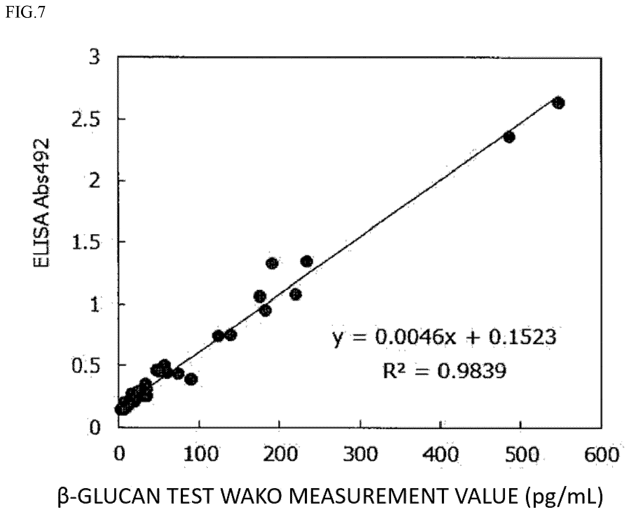

Fig. 7 shows a correlation between the measured value of the Limulus reagent and the absorbance according to ELISA. The absorbance measurement value according to sandwich ELISA of an embodiment of the immunoassay method of the present invention showed a correlation with the measurement value of the Limulus reagent.

### INDUSTRIAL APPLICABILITY

The present invention enables an immunoassay method for BG in a biological sample having a sensitivity equivalent to that of the Limulus reagent. The present invention can provide an assay kit for BG in a biological sample having the sensitivity equivalent to that of the Limulus reagent and easy to operate.

## Claims

1. An immunoassay method for (1->3)-β-D-glucan in a biological sample comprising the steps of:
(i) pretreating the biological sample with an alkali solution;
(ii) neutralising the alkali solution, and
(iii) assaying (1->3)-β-D-glucan in the biological sample by using an anti-(1 ->3)-β-D-glucan monoclonal antibody,
wherein the biological sample is blood, plasma, or serum,
wherein the pH of the alkali solution is 12 or more and the temperature between 4 and 40 °C.

2. The immunoassay method of claim 1, wherein potassium hydroxide (KOH), sodium hydroxide (NaOH) or lithium hydroxide (LiOH) is used in the alkali solution.

3. The immunoassay method according to claims 1 or 2, wherein the pH after neutralisation is 6 to 9.

4. The immunoassay method according to any one of claims 1 to 3, wherein ELISA is used.

5. An assay kit for (1->3)-β-D-glucan in a biological sample comprising following (a) to (c):(a) a solid phase on which a first anti-(1->3)-β-D-glucan monoclonal antibody is immobilized;
(b) an alkali pretreatment solution for the biological sample; wherein the pH of the alkali solution is 12 or more and the temperature between 4 and 40 °C
(c) a solution for neutralization of the alkali pretreatment solution for the biological sample.

6. The assay kit for (1->3)-β-D-glucan in the biological sample according to claim 5, wherein potassium hydroxide (KOH), sodium hydroxide (NaOH) or lithium hydroxide (LiOH) is used in the alkali solution.

7. The assay kit for (1->3)-β-D-glucan in the biological sample according to any one of claims 5 to 6, further comprising (d) a second anti-(1->-3)-β-D-glucan monoclonal antibody labeled with a labeling substance.

8. Use of an alkali pretreatment solution for a biological sample in an immunoassay method for (1->-3)-β-D-glucan in a biological sample using an anti-(1 ->3)-β-D-glucan monoclonal antibody, wherein the pH of the alkali solution is 12 or more and the temperature between 4 and 40 °C.

9. The use of an alkali pretreatment solution for the biological sample according to claim 8, wherein potassium hydroxide (KOH), sodium hydroxide (NaOH) or lithium hydroxide (LiOH) is used.

## Patentansprüche

1. Immunoassay-Verfahren für (1->3)-β-D-Glucan in einer biologischen Probe, umfassend der folgenden Schritte:
(i) Vorbehandeln der biologischen Probe mit einer Alkalilösung;
(ii) Neutralisieren der Alkalilösung, und
(iii) Analysieren von (1->3)-β-D-Glucan in der biologischen Probe unter Verwendung eines Anti-(l->3)-β-D-Glucan monoklonalen Antikörpers,
wobei die biologische Probe Blut, Plasma oder Serum ist, wobei der pH-Wert der Alkalilösung 12 oder mehr und die Temperatur zwischen 4 und 40 °C ist.

2. Immunoassay-Verfahren gemäß Anspruch 1, wobei Kaliumhydroxid (KOH), Natriumhydroxid (NaOH) oder Lithiumhydroxid (LiOH) in der Alkalilösung verwendet wird.

3. Immunoassay-Verfahren gemäß Anspruch 1 oder 2, wobei der pH-Wert nach der Neutralisierung 6 bis 9 beträgt.

4. Immunoassay-Verfahren gemäß irgendeinem der Ansprüche 1 bis 3, wobei ELISA verwendet wird.

5. Assay-Kit für (1->3)-β-D-Glucan in einer biologischen Probe, umfassend die folgenden (a) bis (c):
(a) eine feste Phase, auf der ein erster Anti-(l->3)-β-D-Glucan monoklonalen Antikörpers immobilisiert ist;
(b) eine Alkalivorbehandlungslösung für die biologische Probe, wobei der pH-Wert der Alkalilösung 12 oder mehr und die Temperatur zwischen 4 und 40 °C ist
(c) eine Lösung zur Neutralisierung der Alkalivorbehandlungslösung für die biologische Probe.

6. Assay-Kit für (1->3)-β-D-Glucan in der biologischen Probe gemäß Anspruch 5, wobei Kaliumhydroxid (KOH), Natriumhydroxid (NaOH) oder Lithiumhydroxid (LiOH) in der Alkalilösung verwendet wird.

7. Assay-Kit für (1->3)-β-D-Glucan in der biologischen Probe gemäß Anspruch 5 oder 6, ferner umfassend (d) einen zweiten Anti-(l->3)-β-D-Glucan monoklonalen Antikörper der mit einer Markierungssubstanz markiert ist.

8. Verwendung einer alkalischen Vorbehandlungslösung für eine biologische Probe in einem Immunoassay-Verfahren für (1->-3)-β-D-Glucan in einer biologischen Probe unter Verwendung eines Anti-(l->3)-β-D-Glucan monoklonalen Antikörpers, wobei der pH-Wert der Alkalilösung 12 oder mehr und die Temperatur zwischen 4 und 40 °C ist.

9. Verwendung einer alkalischen Vorbehandlungslösung für die biologische Probe gemäß Anspruch 8, wobei Kaliumhydroxid (KOH), Natriumhydroxid (NaOH) oder Lithiumhydroxid (LiOH) verwendet wird.

## Revendications

1. Procédé de dosage immunologique pour le (1->3)-β-D-glucane dans un échantillon biologique comprenant les étapes consistant à :
(i) prétraiter l'échantillon biologique avec une solution alcaline ;
(ii) neutraliser la solution alcaline, et
(iii) doser le (1->3)-β-D-glucane dans l'échantillon biologique en utilisant un anticorps monoclonal anti-(1->3)-β-D-glucane,
dans lequel l'échantillon biologique est du sang, du plasma ou du sérum,
dans lequel le pH de la solution alcaline est de 12 ou plus et la température entre 4 et 40 °C.

2. Procédé de dosage immunologique selon la revendication 1, dans lequel de l'hydroxyde de potassium (KOH), de l'hydroxyde de sodium (NaOH) ou de l'hydroxyde de lithium (LiOH) est utilisé dans la solution alcaline.

3. Procédé de dosage immunologique selon les revendications 1 ou 2, dans lequel le pH après neutralisation est de 6 à 9.

4. Procédé de dosage immunologique selon l'une quelconque des revendications 1 à 3, dans lequel une ELISA est utilisée.

5. Kit de dosage pour le (1->3)-β-D-glucane dans un échantillon biologique comprenant les (a) à (c) suivants : (a) une phase solide sur laquelle un premier anticorps monoclonal anti-(1→3)-β-D-glucane est immobilisé ;
(b) une solution alcaline de prétraitement pour l'échantillon biologique ; dans lequel le pH de la solution alcaline est de 12 ou plus et la température entre 4 et 40 °C
(c) une solution pour la neutralisation de la solution alcaline de prétraitement pour l'échantillon biologique.

6. Kit de dosage pour le (1->3)-β-D-glucane dans l'échantillon biologique selon la revendication 5, dans lequel de l'hydroxyde de potassium (KOH), de l'hydroxyde de sodium (NaOH) ou de l'hydroxyde de lithium (LiOH) est utilisé dans la solution alcaline.

7. Kit de dosage pour le (1->3)-β-D-glucane dans l'échantillon biologique selon l'une quelconque des revendications 5 à 6, comprenant en outre (d) un second anticorps monoclonal anti-(1->-3)-β-D-glucane marqué avec une substance de marquage.

8. Utilisation d'une solution alcaline de prétraitement pour un échantillon biologique dans un procédé de dosage immunologique pour le (1->-3)-β-D-glucane dans un échantillon biologique utilisant un anticorps monoclonal anti-(1->3)-β-D-glucane, le pH de la solution alcaline étant de 12 ou plus et la température entre 4 et 40 °C.

9. Utilisation d'une solution alcaline de prétraitement pour l'échantillon biologique selon la revendication 8, dans laquelle de l'hydroxyde de potassium (KOH), de l'hydroxyde de sodium (NaOH) ou de l'hydroxyde de lithium (LiOH) est utilisé.
